# EUROPEAN PATENT SPECIFICATION

(11) **EP 1 660 485 B1**
(45) Date of publication and mention of the grant of the patent: **27.12.2006**
(21) Application number: 04743983.1
(22) Date of filing: 12.07.2004
(51) Int. Cl.: C07D 403/06

(54) **IMPROVED PROCESS FOR PREPARING ALPHA-POLYMORPHIC ELETRIPTAN HYDROBROMIDE**
VERBESSERTES VERFAHREN ZUR HERSTELLUNG VON ALPHA-POLYMORPHEM ELETRIPTAN HYDROBROMID
PROCEDE AMELIORE DE PREPARATION D'HYDROBROMURE D'ELETRIPTAN ALPHA-POLYMORPHE

(30) Priority: 23.07.2003 GB 0317229
(43) Date of publication of application: 31.05.2006
(73) Proprietor: Pfizer Limited, Sandwich, Kent CT13 9NJ (GB); Pfizer, Inc., New York, NY 10017 (US)
(72) Inventor: FURLONG, Patrick Joseph, Ringaskiddy API Plant Ringaskiddy,CoCork (IE); KELLY, Cornelius Joseph, Ringaskiddy API Plant Ringaskiddy,CoCork (IE); OGILVIE, Ronald James, Ramsgate Road Sandwich, Kent CT13 9NJ (GB); RYAN, Vincent, Ringaskiddy API Plant Ringaskiddy,CoCork (IE)
(74) Representative: Rudge, Andrew John
(86) International application number: PCT/IB2004/002322
(87) International publication number: WO 2005/007649

(56) References cited:
- WO-A-00/32589
- WO-A-96/06842

## Description

The present invention relates to an improved process for the preparation of the α-polymorphic crystalline form of eletriptan hydrobromide.

Eletriptan, 3-{[1-methylpyrrolidin-2(R)-yl]methyl}-5-(2-phenylsulfonylethyl)-1 H-indole, and a process for its preparation, are disclosed in US-B-5,607,951. Further processes for the preparation of eletriptan are disclosed in EP-B-1088817 and WO-A-02/50063.

Eletriptan hydrobromide has the structure of formula (I) below.

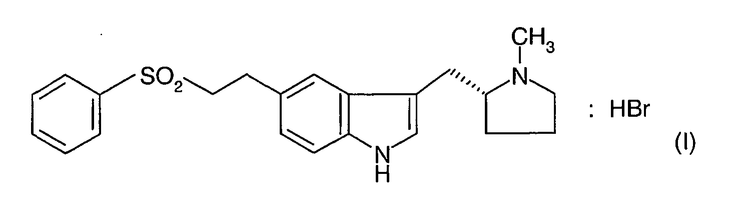

WO-A-96/06842 discloses eletriptan hydrobromide, two of its crystalline forms and processes for the preparation thereof. One of the crystalline forms disclosed therein, designated the α-form, is currently marketed as a treatment for migraine under the name Relpax™.

WO-A-00/32589 discloses a crystalline monohydrate of eletriptan hydrobromide and processes for its preparation.

Two processes for the conversion of eletriptan free base to the α-polymorph of eletriptan hydrobromide are disclosed in WO-A-96/06842. According to the first process, a solution of eletriptan in acetone is treated with an aqueous solution of hydrogen bromide and the resulting oil is crystallised from 2-propanol. According to the second process, a solution of eletriptan in acetone is treated with an aqueous solution of hydrogen bromide and the reaction mixture is slurried, heated at reflux, cooled and slurried a second time.

Under large scale conditions, the yield of eletriptan hydrobromide using these prior art processes is in the region of 73%.

It will be appreciated that in the preparation of sufficient quantities of eletriptan hydrobromide to satisfy the global market for Relpax™, increases in yield, particularly in a late-stage step of the commercial process, are extremely significant in reducing the cost of drug product and are consequently of high commercial importance. Any successful process must also be robust in the sense of reliably producing a homogeneous product containing the same crystalline form, free of other crystalline forms and solvates.

It has now been surprisingly found that a high yielding and robust process for the preparation of the α-polymorph of eletriptan hydrobromide is provided by (a) treating a solution of eletriptan in 2-butanone with hydrobromic acid and (b) distilling off a 2-butanone/water azeotrope until formation of anhydrous α-polymorphic eletriptan hydrobromide is complete.

The yield obtained when using this process on a large scale is in the region of 93 to 96%. The product obtained is exclusively the α-polymorph of eletriptan hydrobromide; no other polymorphic forms or solvates have been observed. Advantageously, the product obtained has a desirable white colouration.

The product of step (a) is thought to be a hydrate, probably the monohydrate described in WO-A-00/32589. This hydrate is then converted in step (b) to form the desired product.

The eletriptan starting material is preferably dry (less than 0.3% water by Karl Fisher analysis) and free of particulate impurities (the solution in 2-butanone can be filtered if necessary). The hydrobromic acid is preferably a 48% aqueous solution and is advantageously added to the reaction vessel as a solution in 2-butanone, over a period of at least an hour and at room temperature. This form of addition ensures that the pH of the reaction mixture does not fall below 5 and leads to a cleaner reaction and a higher yield. The use of from 0.95 to 1.05 molar equivalents of hydrobromic acid is preferred, the use of 0.98 molar equivalents being optimal. About 21 litres of 2-butanone per kilogram of eletriptan starting material should preferably be used in total. After addition of the hydrobromic acid, the reaction is stirred, preferably for a period of at least 3 hours.

During the azeotropic distillation, substantially all the water should be removed from the reaction mixture. A final water content of less than 0.5% weight/weight is preferred. Where about 21 litres of 2-butanone per kilogram of eletriptan starting material has been used in conjunction with 0.98 equivalents of 48% hydrobromic acid, a final volume of about 11 litres per kilogram of eletriptan is ideal.

The product is conveniently isolated by filtration. Typically, the reaction mixture is allowed to cool slowly to room temperature, optionally granulated, filtered, washed with further 2-butanone and dried.

The α-polymorphic eletriptan hydrobromide prepared by the above process may optionally be subjected to a further processing step, known as a polymorph annealing step, which increases its resistance to subsequent hydration. Thus, according to optional step (c), the product of step (b) is slurried in refluxing toluene and a proportion of the toluene is removed by distillation. Preferably, at least 12% of the toluene is removed; most preferably about 16.5% is removed. Step (c) may be optionally repeated.

An initial volume of 15 litres of toluene per kilogram of eletriptan hydrobomide is preferred. For optimal results, the distillation of toluene should be repeated twice and the reaction mixture should be heated at a sub-reflux temperature for a minimum of two hours in between the distillations. A sub-reflux temperature of about 106°C is ideal.
The final product is conveniently isolated by filtration. Typically, the reaction mixture is cooled to room temperature, granulated, filtered, washed with further toluene and dried.

Step (c) is advantageously carried out under an atmosphere of nitrogen to prevent discolouration of the product.

A further embodiment of the invention provides a process for generating stable α-polymorphic eletriptan hydrobromide from any other polymorphic and/or solvated/hydrated form of eletriptan hydrobromide or from a mixture of different polymorphic and/or solvated/hydrated forms (including a mixture comprising the α-polymeric form itself).

This conversion process comprises the steps of (a) crystallising a solution of the eletriptan hydrobromide starting material in a mixture of 2-butanone and water and (b) distilling off a 2-butanone/water azeotrope until formation of anhydrous α-polymorphic eletriptan hydrobromide is complete. An optional annealing step (c), as described above, may also be carried out.

This process is particularly advantageous since previously the only viable large scale process for converting mixed polymorphic and/or solvated/hydrated forms of eletriptan hydrobromide to the pure α-polymorph of eletriptan hydrobromide involved breaking the salt to the free base as a preliminary step.

Eletriptan is preferably prepared according to Scheme 1 below.

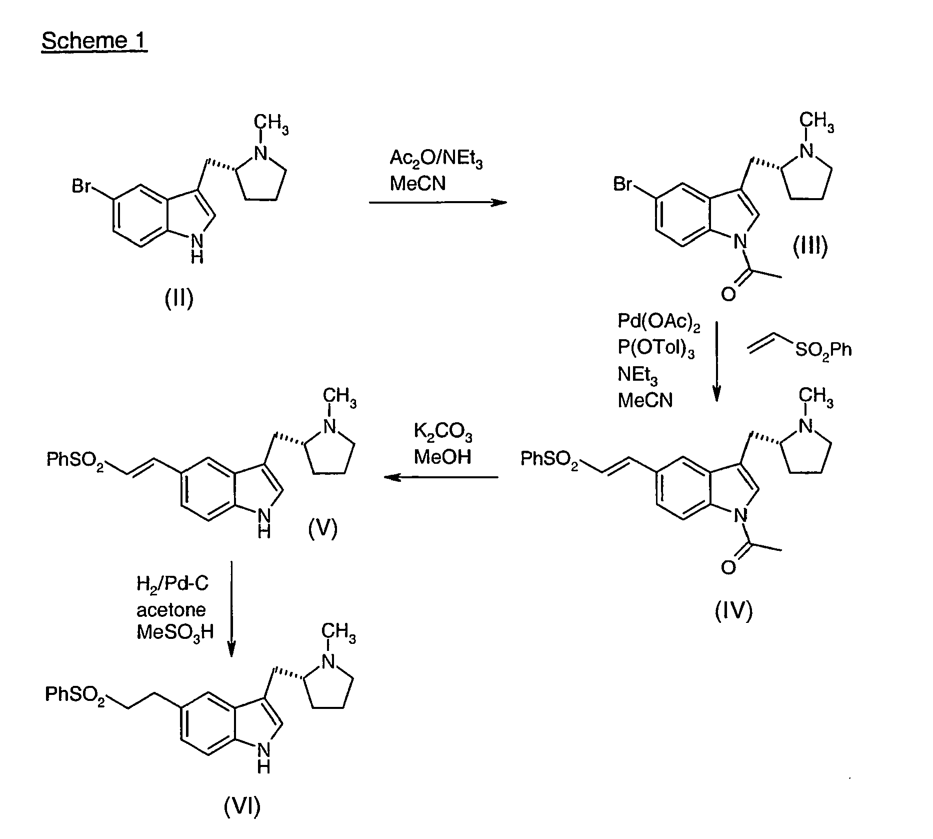

Compound (II) ((R)-5-bromo-3-(N-methylpyrrolidine-2-ylmethyl)-1H-indole) may be prepared by the methods described in US-B-5,607,951 or EP-B-1088817.

Compound (III) ((R)-1-acetyl-5-bromo-3-(N-methylpyrrolidine-2-ylmethyl)-1 H-indole) may be prepared by treating a solution of compound (II) in acetonitrile, with acetic anhydride and triethylamine. The reaction is preferably carried out under reflux.

Compound (IV) ((R)-1-acetyl-5-(2-phenylsulphonylethenyl)-3-(N-methylpyrrolidin-2-ylmethyl)-1H-indole) may be prepared by treating a solution of compound (III) in acetonitrile with triethylamine, tri-o-tolylphosphine, palladium acetate and phenylvinylsulphone. The reaction is preferably carried out under reflux.

Conveniently, compound (II) may be converted to compound (IV) without isolation of compound (III).

Compound (V) ((R)-5-(2-phenylsulphonylethenyl)-3-(N-methylpyrrolidin-2-ylmethyl)-1 H-indole) may be prepared by treating a solution of compound (IV) in methanol with potassium carbonate. The reaction is preferably carried out at room temperature. A similar process, described in the prior art (see Example 58 of US-B-5,607,951) uses recrystallisation to purify and isolate compound (V). Recrystallisation is indeed an effective means of purification, particularly from a mixture of acetonitrile and water or a mixture of acetone and water, but the yield of product obtained is sub-optimal. Surprisingly, it has been found that column chromatography is a more efficient method of purifying crude compound (V), even on a large multikilogram scale, and a significantly higher yield of product can be isolated in this way.

When chromatography is used to purify compound (V), the methanolic solution resulting from the reaction is filtered and neutralised with an aqueous acid, preferably phosphoric acid. The mixture is then loaded onto a column packed with a suitable stationary phase (preferably a styrene polymer such as CG-161 resin, available from Tosoh Bioscience). The column is eluted with a mixture of acetone and an aqueous acid (preferably acetic or phosphoric acid) and the fractions containing product are combined and concentrated. Acetone is added to the concentrated solution and the pH is adjusted to from 10 to 11 using a suitable base, such as potassium carbonate, to precipitate the product. The product is collected, washed with water and dried.

Compound (VI) (eletriptan) may be prepared by treating a solution of compound (V) in a mixture of acetone and water with palladium on carbon and methanesulphonic acid under an atmosphere of hydrogen. A catalyst comprising 5% palladium on carbon is preferred. A particularly advantageous catalyst for use in this process is PMC 2020C (supplied by the Precious Metals Corporation), requiring a catalyst loading as low as 7%.

The following Examples illustrate particular ways of putting the invention into effect. Differential scanning calorimetry (DSC) was performed using a Perkin Elmer DSC-7 instrument. Approximately 10mg of each sample was accurately weighed into a 50 microlitre aluminium pan. The samples were heated at 20°C/minute over the range 40°C to 220°C with a nitrogen gas purge.

### Example 1

(a) (R)-5-Bromo-3-(N-methylpyrrolidine-2-ylmethyl)-1H-indole (256kg), acetonitrile (380kg), triethylamine (115kg) and acetic anhydride (115kg) were charged to a dry glass lined vessel. The reaction mixture was heated to reflux and maintain at this temperature for 4.5 hours.
(b) A mixture of acetonitrile (375kg), palladium acetate (12.5kg) and tri-o-tolylphosphine (60kg) was stirred for 1 hour. Phenyl vinyl sulphone (160kg), triethylamine (92kg) and finally the solution prepared in part (a) were added and the mixture was heated to reflux for 7.5 hours. The reaction mixture was cooled and a solution of 190kg concentrated hydrochloric acid in 1200kg water was added over 4 hours. The resulting mixture was filtered to remove spent catalyst and a further 3000kg of water and 300kg of 50% w/w aqueous sodium hydroxide solution were added to the filtrate to precipitate the product. The resulting suspension was filtered and washed with water (500kg) to yield crude (R)-1-acetyl-5-(2-phenylsulphonylethenyl)-3-(N-methylpyrrolidin-2-ylmethyl)-1H-indole) as a dark brown, wet, crystalline solid (535kg wet, equivalent of 338kg dry). This crude product was added to 530kg of acetone and the mixture was heated to 60°C. On reaching this temperature 814kg of water was added over 2 hours whilst simultaneously cooling the mixture back to ambient temperature. The batch was then granulated for 2 hours and filtered to yield the purified product (350kg wet, equivalent of 280kg dry, 83%).

### Example 2

Methanol (660kg) and (R)-1-acetyl-5-(2-phenylsulphonylethenyl)-3-(N-methylpyrrolidin-2-ylmethyl)-1H-indole (77kg dry equivalent of the recrystallised product of Example 1) were charged to a vessel and the resulting mixture was stirred for 5 minutes. Potassium carbonate (8.9kg) was added and the mixture was stirred at room temperature for 30minutes. The reaction mixture was then warmed to 35°C and Norit carbon (11.6kg) and water (235kg) were added. The resulting mixture was filtered and the filtrate was diluted by the addition of water (1300kg, added over two hours) and granulated for 2 hours at room temperature. Filtration gave crude (R)-5-(2-phenylsulphonylethenyl)-3-(N-methylpyrrolidin-2-ylmethyl)-1H-indole (75kg wet, equivalent of 60.5kg dry, 88%).

### Example 3

A mixture of acetonitrile (940kg) and crude (R)-5-(2-phenylsulphonylethenyl)-3-(N-methylpyrrolidin-2-ylmethyl)-1 H-indole (478kg dry equivalent, product of the process of Example 2) was warmed to 55°C. Water (720kg) was added and the mixture was cooled to 20°C and granulated for 2 hours at that temperature. Pure (R)-5-(2-phenylsulphonylethenyl)-3-(N-methylpyrrolidin-2-ylmethyl)-1H-indole (482kg wet, equivalent of 393kg dry, 82%) was recovered by filtration.

### Example 4

Acetone (1140kg) and (R)-5-(2-phenylsulphonylethenyl)-3-(N-methylpyrrolidin-2-ylmethyl)-1H-indole (482kg dry equivalent, recrystallised product of Example 3) were charged to a vessel and the mixture was warmed to 55°C. Water (1520kg) was added and the mixture was cooled to 20°C and granulated for 2 hours. Recrystallised (R)-5-(2-phenylsulphonylethenyl)-3-(N-methylpyrrolidin-2-ylmethyl)-1H-indole (492kg wet, 343kg dry equivalent, 87%) was isolated by filtration.

### Example 5

(R)-5-(2-Phenylsulphonylethenyl)-3-(N-methylpyrrolidin-2-ylmethyl)-1H-indole (200kg dry equivalent) and acetone (1186.5kg) were charged to a dry, glass lined vessel. De-ionised water (300kg), further acetone (237kg), methanesulphonic acid (55kg) and a slurry of palladium on carbon (22.4kg dry equivalent) in de-ionised water (200 kg) were added and the mixture was hydrogenated under an atmosphere of hydrogen gas. The reaction slurry was filtered to remove the catalyst. De-ionised water (1300kg) and 48% aqueous sodium hydroxide solution (60 kg) were added to precipitate the product, which was isolated by filtration and washed with a mix of de-ionised water (210kg), acetone (83kg) and further de-ionised water (710kg) to yield 3-{[1-methylpyrrolidin-2(R)-yl]methyl}-5-(2-phenylsulphonylethyl)-1H-indole (220kg wet, 157.65kg when dry, 78.41%).

### Example 6

3-{[1-Methylpyrrolidin-2(R)-yl]methyl}-5-(2-phenylsulphonylethyl)-1H-indole (15kg) and 2-butanone (204kg) were charged to a dry, glass-lined vessel. A solution of 48% aqueous hydrobromic acid (6.45kg) in 2-butanone (63kg) was added and the resulting slurry was subjected to azeotropic distillation until a volume of 150 litres remained. The reaction mixture was cooled to 17.5°C and the product was isolated by filtration. The product was washed with 2-butanone (16kg) to yield the α-polymorphic form of 3-{[1-Methylpyrrolidin-2(R)-yl]methyl}-5-(2-phenylsulphonylethyl)-1 H-indole hydrobromide (18.2kg wet, 17.5 kg when dry, 96.3%).
- DSC:: A single major endotherm with a peak maximum in the range 173°-179°C was observed, indicative of the α-polymorph (see WO-A-96/06842).

### Example 7

α-Polymorphic 3-{[1-methylpyrrolidin-2(R)-yl]methyl}-5-(2-phenylsulphonylethyl)-1 H-indole hydrobromide (300kg) and toluene (3892kg) were charged to a dry, glass-lined vessel. The resulting slurry was heated under reflux and approximately 666kg of toluene was removed by distillation. The slurry was cooled to 100-105°C and then a further 666kg of toluene was removed by distillation. The reaction slurry was then cooled to 22.5°C and the product was isolated by filtration. The product was washed with toluene (908kg) to yield α-polymorphic 3-{[1-methylpyrrolidin-2(R)-yl]methyl}-5-(2-phenylsulphonylethyl)-1 H-indole hydrobromide (289 kg dry weight, 96.3%).
- DSC:: A single major endotherm with a peak maximum in the range 173°-179°C was observed, indicative of the α-polymorph (see WO-A-96/06842).

### Example 8

3-{[1-Methylpyrrolidin-2(R)-yl]methyl}-5-(2-phenylsulphonylethyl)-1 H-indole hydrobromide (10kg), 2-butanone (63kg) and de-ionised water (0.65kg) were charged to a dry, glass-lined vessel and heated to 67.5°C to form a solution. The solution was then cooled to 60°C and further 2-butanone (42 kg) was added to precipitate the product. The resulting slurry was subjected to an azeotropic distillation to leave a final reaction volume of 50 litres and then cooled to 22.5 °C. The product was isolated by filtration and washed with 2-butanone (10.5kg) to yield α-polymorphic 3-{[1-methylpyrrolidin-2(R)-yl]methyl}-5-(2-phenylsulphonylethyl)-1H-indole hydrobromide (9.3kg dry weight, 93%).
- DSC:: A single major endotherm with a peak maximum in the range 173°-179°C was observed, indicative of the α-polymorph (see WO-A-96/06842).

### Comparative example

3-{[1-Methylpyrrolidin-2(R)-yl]methyl}-5-(2-phenylsulphonylethyl)-1H-indole (274kg) and acetone (3419kg) were charged to a dry, glass-lined vessel. A solution of 48% aqueous hydrobromic acid (114.7kg) in acetone (1383kg) was added at a temperature of from 50 to 55°C over 1 hour and the resulting slurry was stirred for 4 hours. The reaction mixture was cooled to from 30 to 35°C and the product was isolated by filtration. The product was washed with acetone (861 kg) to yield the α-polymorphic form of 3-{[1-methylpyrrolidin-2(R)-yl]methyl}-5-(2-phenylsulphonylethyl)-1 H-indole hydrobromide (242.4 kg dry, 73.0%).
- DSC:: A single major endotherm with a peak maximum in the range 173°-179°C was observed, indicative of the α-polymorph (see WO-A-96/06842).

## Claims

1. A process for preparing α-polymorphic eletriptan hydrobromide comprising the steps of (a) treating a solution of eletriptan in 2-butanone with hydrobromic acid and (b) distilling off a 2-butanone/water azeotrope until formation of anhydrous α-polymorphic eletriptan hydrobromide is complete.

2. A process as claimed in claim 1 wherein the water content of the reaction mixture is reduced to less than 0.5% weight/weight in step (b).

3. A process as claimed in claim 1 or claim 2 comprising the extra step of slurrying the product of step (b) in refluxing toluene and removing a proportion of the toluene by distillation.

4. A process as claimed in claim 3 wherein at least 12% of the toluene is removed.

5. A process as claimed in any one of the preceding claims wherein the eletriptan starting material is prepared by treating a solution of (R)-5-(2-phenylsulphonylethenyl)-3-(N-methylpyrrolidin-2-ylmethyl)-1H-indole in acetone with palladium on carbon and methanesulphonic acid under an atmosphere of hydrogen.

6. A process as claimed in claim 5 wherein the (R)-5-(2-phenylsulphonylethenyl)-3-(N-methylpyrrolidin-2-ylmethyl)-1H-indole is prepared by treating a solution of (R)-1-acetyl-5-(2-phenylsulphonylethenyl)-3-(N-methylpyrrolidin-2-ylmethyl)-1H-indole in methanol with potassium carbonate.

7. A process as claimed in claim 6 wherein the (R)-5-(2-phenylsulphonylethenyl)-3-(N-methylpyrrolidin-2-ylmethyl)-1 H-indole is purified by column chromatography.

8. A process as claimed in claim 6 or claim 7 wherein the (R)-1-acetyl-5-(2-phenylsulphonylethenyl)-3-(N-methylpyrrolidin-2-ylmethyl)-1H-indole is prepared by treating a solution of (R)-1-acetyl-5-bromo-3-(N-methylpyrrolidine-2-ylmethyl)-1 H-indole in acetonitrile with triethylamine, tri-o-tolylphosphine, palladium acetate and phenylvinylsulphone.

9. A process as claimed in claim 8 wherein the (R)-1-acetyl-5-bromo-3-(N-methylpyrrolidine-2-ylmethyl)-1H-indole is prepared by treating a solution of (R)-5-bromo-3-(N-methylpyrrolidine-2-ylmethyl)-1H-indole in acetonitrile with acetic anhydride and triethylamine.

10. A process for preparing a crystalline, α-polymorphic form of eletriptan hydrobromide from any other polymorphic and/or solvated/hydrated form of eletriptan hydrobromide or from a mixture of different polymorphic and/or solvated/hydrated forms, including a mixture comprising the α-polymeric form itself, comprising (a) crystallising a solution of the eletriptan hydrobromide starting material in a mixture of 2-butanone and water and (b) distilling off a 2-butanone/water azeotrope until the formation of anhydrous α-polymorphic eletriptan hydrobromide is complete.

11. A process as claimed in claim 10 comprising the extra step of slurrying the product of step (b) in refluxing toluene and removing a proportion of the toluene by distillation.

## Patentansprüche

1. Verfahren zur Herstellung von α-polymorphem Eletriptan-Hydrobromid, umfassend die Schritte:
(a) Behandeln einer Lösung von Eletriptan in 2-Butanon mit Bromwasserstoffsäure und
(b) Abdestillieren eines 2-Butanon/Wasser-Azeotrops, bis die Bildung von wasserfreiem α-polymorphem Eletriptan-Hydrobromid vollständig ist.

2. Verfahren nach Anspruch 1, wobei der Wassergehalt des Reaktionsgemisches in Schritt (b) auf weniger als 0,5% Gewicht/Gewicht reduziert wird.

3. Verfahren nach Anspruch 1 oder Anspruch 2, das den zusätzlichen Schritt eines Aufschlämmens des Produktes von Schritt (b) in refluxierendem Toluol und Entfernen eines Verhältnisanteils des Toluols durch Destillation umfasst.

4. Verfahren nach Anspruch 3, wobei wenigstens 12% des Toluols entfernt werden.

5. Verfahren nach einem der vorangehenden Ansprüche, wobei das Eletriptan-Ausgangsmaterial durch Behandeln einer Lösung von (R)-5-(2-Phenylsulfonylethenyl)-3-(N-methylpyrrolidin-2-ylmethyl)-1 H-indol in Aceton mit Palladium-auf-Kohle und Methansulfonsäure unter Wasserstoffatmosphäre hergestellt wird.

6. Verfahren nach Anspruch 5, wobei das (R)-5-(2-Phenylsulfonylethenyl)-3-(N-methylpyrrolidin-2-ylmethyl)-1 H-indol hergestellt wird, indem eine Lösung von (R)-1-Acetyl-5-(2-phenylsulfonylethenyl)-3-(N-methylpyrrolidin-2-ylmethyl)-1 H-indol in Methanol mit Kaliumcarbonat behandelt wird.

7. Verfahren nach Anspruch 6, wobei das (R)-5-(2-Phenylsulfonylethenyl)-3-(N-methylpyrrolidin-2-ylmethyl)-1 H-indol durch Säulenchromatographie gereinigt wird.

8. Verfahren nach Anspruch 6 oder Anspruch 7, wobei das (R)-1-Acetyl-5-(2-phenylsulfonylethenyl)-3-(N-methylpyrrolidin-2-ylmethyl)-1 H-indol hergestellt wird, indem eine Lösung von (R)-1-Acetyl-5-brom-3-(N-methylpyrrolidin-2-ylmethyl)-1 H-indol in Acetonitril mit Triethylamin, Tri-o-tolylphosphin, Palladiumacetat und Phenylvinylsulfon behandelt wird.

9. Verfahren nach Anspruch 8, wobei das (R)-1-Acetyl-5-brom-3-(N-methylpyrrolidin-2-ylmethyl)-1 H-indol hergestellt wird, indem eine Lösung von (R)-5-Brom-3-(N-methylpyrrolidin-2-ylmethyl)-1 H-indol in Acetonitril mit Essigsäureanhydrid und Triethylamin behandelt wird.

10. Verfahren zur Herstellung einer kristallinen, α-polymorphen Form von Eletriptan-Hydrobromid aus einer anderen polymorphen und/oder solvatisierten/hydratisierten Form von Eletriptan-Hydrobromid oder aus einem Gemisch verschiedener polymorpher und/oder solvatisierter/hydratisierter Formen, einschließlich eines Gemisches, das die α-polymere Form selbst umfasst, umfassend:
(a) Kristallisieren einer Lösung des Eletriptan-Hydrobromid-Ausgangsmaterials in einem Gemisch aus 2-Butanon und Wasser und
(b) Abdestillieren eines 2-Butanon/Wasser-Azeotrops, bis die Bildung von wasserfreiem, α-polymorphem Eletriptan-Hydrobromid vollständig ist.

11. Verfahren nach Anspruch 10, das den zusätzlichen Schritt eines Aufschlämmens des Produkts von Schritt (b) in refluxierendem Toluol und Entfernen eines Verhältnisanteils des Toluols durch Destillation umfasst.

## Revendications

1. Procédé pour la préparation de bromhydrate d'élétriptan polymorphe α, comprenant les étapes consistant (a) à traiter une solution d'élétriptan dans de la 2-butanone avec de l'acide bromhydrique et (b) à éliminer par distillation un azéotrope 2-butanone/eau jusqu'à ce que la formation de bromhydrate d'élétriptan polymorphe α anhydre soit achevée.

2. Procédé suivant la revendication 1, dans lequel la teneur en eau du mélange réactionnel est réduite à moins de 0,5 % en poids/poids dans l'étape (b).

3. Procédé suivant la revendication 1 ou la revendication 2, comprenant l'étape supplémentaire de mise en suspension du produit de l'étape (b) dans du toluène au reflux et d'élimination d'une proportion du toluène par distillation.

4. Procédé suivant la revendication 3, dans lequel au moins 12 % du toluène sont éliminés.

5. Procédé suivant l'une quelconque des revendications précédentes, dans lequel l'élétriptan servant de matière de départ est préparé en traitant une solution de (R)-5-(2-phénylsulfonyléthényl)-3-(N-méthylpyrrolidine-2-ylméthyl)-1H-indole dans de l'acétone avec du palladium sur du carbone et de l'acide méthanesulfonique sous atmosphère d'hydrogène.

6. Procédé suivant la revendication 5, dans lequel le (R)-5-(2-phénylsulfonyléthényl)-3-(N-méthylpyrrolidine-2-ylméthyl)-1H-indole est préparé en traitant une solution de (R)-1-acétyl-5-(2-phénylsulfonyléthényl)-3-(N-méthylpyrrolidine-2-ylméthyl)-1H-indole dans du méthanol avec du carbonate de potassium.

7. Procédé suivant la revendication 6, dans lequel le (R)-5-(2-phénylsulfonyléthényl)-3-(N-méthylpyrrolidine-2-ylméthyl)-1H-indole est purifié par chromatographie sur colonne.

8. Procédé suivant la revendication 6 ou la revendication 7, dans lequel le (R)-1-acétyl-5-(2-phénylsulfonyléthényl)-3-(N-méthylpyrrolidine-2-ylméthyl)-1H-indole est préparé en traitant une solution de (R)-1-acétyl-5-bromo-3-(N-méthylpyrrolidine-2-ylméthyl)-1H-indole dans de l'acétonitrile avec de la triéthylamine, de la tri-o-tolylphosphine, de l'acétate de palladium et de la phénylvinylsulphone.

9. Procédé suivant la revendication 8, dans lequel le (R)-1-acétyl-5-bromo-3-(N-méthylpyrrolidine-2-ylméthyl)-1H-indole est préparé en traitant une solution de (R)-5-bromo-3-(N-méthylpyrrolidine-2-ylméthyl)-1H-indole dans de l'acétonitrile avec de l'anhydride acétique et de la triéthylamine.

10. Procédé pour la préparation d'une forme polymorphe α cristalline de bromhydrate d'élétriptan à partir de n'importe quelle autre forme polymorphe et/ou solvatée/hydratée de bromhydrate d'élétriptan ou à partir d'un mélange de différentes formes polymorphes et/ou solvatées/hydratées, y compris un mélange comprenant la forme polymorphe α proprement dite, comprenant (a) la cristallisation d'une solution du bromhydrate d'élétriptan servant de matière de départ dans un mélange de 2-butanone et d'eau et (b) l'élimination par distillation d'un azéotrope 2-butanone/eau jusqu'à ce que la formation de bromhydrate d'élétriptan polymorphe α anhydre soit achevée.

11. Procédé suivant la revendication 10, comprenant l'étape supplémentaire de mise en suspension du produit de l'étape (b) dans du toluène au reflux et d'élimination d'une proportion du toluène par distillation.
